# EUROPEAN PATENT APPLICATION

(11) **EP 1 645 255 A1**
(43) Date of publication of application: **12.04.2006**
(21) Application number: 04445108.6
(22) Date of filing: 11.10.2004
(51) Int. Cl.: A61F 13/08, A61H 23/02

(54) **Electro active compression bandage**

(71) Applicant: SMM Medical AB, 582 16 Linköping (SE)
(72) Inventor: Larson, Tomas, 590 55 Sturefors (SE); Toth, Landy, 582 18 Linköping (SE)
(74) Representative: Wihlsson, Joakim Per Magnus

(57) **Abstract**

The invention relates to exertion of an external pressure to a human body part (100). A proposed device includes a number of segments (S1, S2, S3) adapted to at least partially enclose the body part (100) in a form-fitting manner. Each segment (S1, S2, S3) is adapted to at least partially enclose the body part (100) in a form-fitting manner and apply an adjustable pressure profile to the body part (100). The pressure profile is produced in direct response to an electrical control signal (C(i)) by means of an electroactive-material-based actuator (A) in each segment (S1, S2, S3).

## Description

### THE BACKGROUND OF THE INVENTION AND PRIOR ART

The present invention relates generally to the application of pressure profiles to living tissues. More particularly the invention relates to a device for exerting an external pressure to a human body part according to the preamble of claim 1 and a therapeutic garment according to the preamble of claim 21.

External pressure profiles may be applied to living tissues, e.g. of a person's limb, in order to attain various effects with respect to these tissues. Perhaps the most well known example is the so-called G-suit worn by a pilot to restrict the blood circulation in his/her lower body parts under certain conditions, and thus reduce the risk that an insufficient amount of blood is fed to the pilot's head.

However, also in the medical field there are many examples of situations in which it is relevant/desirable to apply an external pressure to a part of the human body, in order to cure or mitigate a disease or condition. For instance, lymphoedema is a condition where the lymphatic system of a patient has been compromised, thereby resulting in a buildup of lymphatic fluids and proteins in one or more extremities. So far, various approaches have been attempted to control the swelling of the afflicted extremities. The compression-based treatment of lymphoedema is primarily dealt with in three ways, which may be combined to achieve an improved result, compression bandaging, pneumatic compression pumps and massage. The compression bandaging may be further divided into two general approaches: multi-layered lymphatic bandaging and elastic compression garments/ bandages. Both these methods are used to statically compress the afflicted limbs, whereas pneumatic compression pumps and massage represent dynamic treatments.

Multi-layered lymphatic bandages are applied to a patient in order to reshape one or more of the patient's limbs. The bandages consist of absorbent layers, padding and short-stretch bandages. The absorbent layers must be custom made by a technician to fit the patient. Moreover, the underlying pressure is unknown after application, and the bandages are there to prevent the limb from further expanding and to breakup proteins with the help of patient movement. Nevertheless, these bandages are associated with numerous problems. During the treatment the bandages must be adjusted many times, for example because the bandages have a static shape and the shape of the limb varies over time. The bandages are also bulky and hot to wear due to the many layers applied to the limb, and therefore the bandages cannot be worn under clothing. Naturally, the therapy for the patient is limited in that the multi-layered lymphatic bandages cannot actively pressurize the body.

Elastic compression bandages are used to statically pressurize an afflicted limb. Here, a caregiver wraps the afflicted limb with a combination of elastic bandages and absorbent layers. The bandages are arranged so as to apply a graduated pressure to the limb. The pressure gradient along the limb is structured such that the highest pressure is at the distal end, and the lowest pressure is located at the proximal end of the limb. Hence, also in this case, the pressure application is static and a qualified person must apply the bandages to ensure that an appropriate pressure is accomplished, particularly since there is no convenient way to accurately measure the pressure applied to the limb. Normally, a constant bandage tension is applied while wrapping the limb, and the pressure graduation is typically a consequence of the limb being thinner at the distal part than at the proximal part. As the limb changes size due to the pressure, and as the bandages creep, the pressure application will decrease. This is true already within hours of applying the bandages.

A pneumatic compression pump device is used to dynamically pressurize limbs of patients. Here, dynamic pressurization is employed both to pump lymphatic fluids from the limb in wavelike, or graduated, pressure profiles and to breakup proteins that collect and harden in the afflicted limb. To generate the wavelike and graduated pressure profiles along the limb, a sleeve portion of the device must have multiple chambers. Each chamber is pressurized at the appropriate time as determined by the treatment prescription. For instance U.S. patent No. 6,436,064 discloses a compression garment of this type, which may be used for treatment of lymphoedema. However, the pneumatic compression pump devices are relatively inefficient, and therefore cannot operate from batteries for any significant length of time. In fact, it is normally required that the device be connected to mains power, and as a further consequence that the patient be stationary during the treatment. Since the pneumatic compression pump device is airtight, heat produced by the patient is accumulated in the device. Thus, the device can only be used for comparatively short durations before it becomes too uncomfortable for the patient. Although the device can dramatically reduce oedema during treatment, after use, static compression bandages (or equivalent) must be applied to prevent the fluids from draining back into the afflicted limb. Additionally, the device is noisy, the air-pressure measurements used to infer pressure applied to the limb can be inaccurate, and unintentionally high pressure levels may harm the patient.

A qualified massage therapist/clinician may also apply various forms of massage to a patient. Such massage techniques are highly technical and require significant training to perform. Thus, the outcome of the treatment depends very much on the skill of the clinician.

U.S. patent No. 5,997,465 describes a device for exerting an external pressure on a human body, wherein the device surrounds a body part with a comfortable fit. The device includes memory material components, which alter their shape in response to an electrical signal. Thereby, in a contracted state, these components may squeeze the body part, for example to prevent pooling of blood in the body part of a pilot when subjected to G-forces. Then, in a non-contracted state (i.e. when no electrical signal is present) the memory material components resume their original shape, and the squeezing ceases.

The electrically controllable memory material components proposed in this document overcome many of the shortcomings associated with the above-described dynamic procedures, i.e. the pneumatic compression pump devices and massage forms. However, since the memory materials undergo phase transformations at given temperatures, heating and cooling are used as control parameters to the pressure device. Naturally, an electrical signal may produce the heating necessary to accomplish activation of an actuator. On the other hand, deactivation of the actuator depends on a cooling rate, which in turn, is a function highly correlated with the temperatures and cooling rates of any surrounding materials, which in medical applications would include various body parts. It is therefore very difficult to finely control memory-based material actuators, since this requires controlling the material in fine bands around the transition temperatures. Also the speed of response is hard to control. Furthermore, since the temperature constitutes the link between the electrical control signal and the desired mechanical output, significant amounts of energy are lost in the electrothermo-to-thermomechanical coupling. In medical applications, such losses are especially undesired. Namely, to maximize patient mobility, it is often demanded that medical devices be battery operated (and thus power efficient). It is also important that the patient's temperature can be regulated accurately, since high temperatures cannot be tolerated for prolonged periods.

### SUMMARY OF THE INVENTION

The object of the present invention is therefore to alleviate the above-mentioned problems and thus accomplish improved pressure profiles with respect to controllability, efficiency and medical suitability.

According to one aspect of the invention, the object is achieved by the initially described device, wherein each segment contains an electroactive-material-based actuator, which is adapted to produce the adjustable pressure profile in direct response to an electrical control signal.

An important advantage attained thereby is that an efficient pressure control can be attained, which is accurate in terms of both timing and pressure level. The high efficiency of the proposed device renders the design particularly advantageous in cases where a high duty cycle is required, or an activated state must be maintained for long periods of time.

According to one preferred embodiment of this aspect of the invention, each of the actuators has a morphology which is adjustable by the electrical control signal, and a change of the morphology is instigated by the electrical control signal. Moreover, each of the actuators is adapted to maintain a thus changed morphology on the basis of an electrical control signal supplying charge replenishment to the actuator. Consequently, it is practically only necessary to add or remove charges when adjusting an actuator. The charge replenishment represents very small quantities of energy that compensate for charge leakage due to minor conductive effects in the electroactive-material.

According to another preferred embodiment of this aspect of the invention, at least one of the actuators is adapted to convert mechanical energy produced by the body part into electric energy. Hence, the device user may contribute to the powering of the device. Namely, for instance in contrast to memory materials, the direct electromechanical coupling of the proposed electroactive material is reversible.

According to yet another preferred embodiment of this aspect of the invention, at least one of the segments includes an actuator that has an interface towards a pressure transition system. This system, in turn, is adapted to redistribute a basic pressure profile of the actuator, such that in response to a control signal in respect of the segment, an adjusted pressure profile different from the basic pressure profile is applied to the body part. Thereby, a smoother (a more fuzzy) pressure profile and/or a pressure profile which is adapted to treat a particular condition can be applied to the body part.

According to other preferred embodiments of this aspect of the invention, at least one of the actuators includes an electroactive material in the form of an electroactive polymer or ceramic, which is either a field activated electroactive material adapted to operate based on Maxwell stress effects, electrostrictive or piezoelectric effects, or an ionic electroactive material, for example an ion-exchange polymer metal composite material, a conducting polymer or a gel-based electrolyte. Videlicet, due to their intrinsic characteristics, all these materials enable very compact and slim device designs that are suitable for many purposes, particularly within the medical field.

According to another preferred embodiment of this aspect of the invention, at least one of the actuators includes one or more flexible interfaces towards the segment to which the actuator is associated. This interface is adapted to link mechanical energy produced the actuator towards the body part, and thereby improves the actuator's overall effectiveness.

According to yet another preferred embodiment of this aspect of the invention, the device includes a control unit adapted to apply control signals to the segments. The control unit varies the control signals over time to implement a treatment profile with respect to the body part. The treatment profile may involve applying gradually varying pressure profiles to the body part via the segments, for example in the form of repeated cycles of variations between relatively high and relatively low basic pressure profiles. Thereby, many different medical conditions can be treated successfully.

According to still another preferred embodiment of this aspect of the invention, at least one of the actuators includes a sensor element adapted to register a physiological parameter of the body part and/or an environmental condition in proximity to the body part, and generate a data signal that reflects this parameter. Preferably, the control unit receives the data signal and adjusts the treatment profile in response to the data signal. Consequently, the treatment profile can be made dependant on a current state of the body part and/or the current environmental conditions. Moreover, one or more data signals may reflect a patient's posture. Thus, it is also possible to adapt the treatment profile to a current posture.

According to another preferred embodiment of this aspect of the invention, the sensor element constitutes a subdivision of the actuator. Hence, the intrinsic sensory capabilities of the actuating material are used to generate a relevant data signal, which in turn, may enhance the treatment of a patient.

According to another aspect of the invention, the object is achieved by the initially described therapeutic garment, wherein the garment includes at least one of the proposed devices. Of course, such a garment is advantageous for the same reasons as the above-described device.

To sum up, the invention presents an efficient and highly flexible solution for applying an external pressure to a human body part. Thereby, the invention may be optimized for various medical purposes, and for instance be used for treatment or prophylaxis of lymphoedema, deep venous thrombosis, venous leg ulcers, venous insufficiency, arterial ulcers, arterial insufficiency, diabetic foot ulcers, cardiovascular diseases, claudication, burns and sports injuries. The proposed solution may also be used in stress therapy, massage therapy, enhanced external counter pulsation therapy and blood pressure monitoring.

Further advantages, advantageous features and applications of the present invention will be apparent from the following description.

### BRIEF DESCRIPTION OF THE DRAWINGS

The present invention is now to be explained more closely by means of preferred embodiments, which are disclosed as examples, and with reference to the attached drawings.
- Figures 1a-b: illustrate a basic morphology of a planar field activated EAM-based actuator according to one embodiment of the invention,
- Figures 2a-c: illustrate the morphology of a cylindrical field activated EAM-based actuator according to one embodiment of the invention,
- Figures 3a-b: illustrate the morphology of a field activated EAM-based actuator of multilayer type according to one embodiment of the invention,
- Figures 4a-b: illustrate the morphology of a field activated EAM-based actuator of C-block type according to one embodiment of the invention,
- Figures 5a-b: illustrate the morphology of a field activated EAM-based actuator of bubble type according to one embodiment of the invention,
- Figure 6: shows a field activated EAM-based actuator of a first cymbal type according to one embodiment of the invention,
- Figures 7a-b: show a field activated EAM-based actuator of a second cymbal type having a flexible interface to link mechanical energy produced the actuator towards a body part according to one embodiment of the invention,
- Figures 8a-b: illustrate a basic morphology of an ionic EAM-based actuator according to one embodiment of the invention,
- Figures 9a-b: illustrate the morphology of a bilayer ionic EAM-based actuator according to one embodiment of the invention,
- Figures 10a-b: illustrate the morphology of a triple ionic layer EAM-based actuator according to one embodiment of the invention,
- Figures 11a-b: illustrate the morphology of an ion-exchange polymer metal composite EAM-based actuator according to one embodiment of the invention,
- Figure 12: shows an EAM-based actuator including an ion-exchange gel according to one embodiment of the invention,
- Figures 13a-b: illustrate the morphology of an EAM-based actuator including an ion-exchange gel according to one embodiment of the invention,
- Figures 14a-c: show side and top views of a conducting polymer actuator according to one embodiment of the invention,
- Figures 15a-b: schematically illustrate the operation of a segment in a device according to one embodiment of the invention which includes actuators of the type shown in the figures 14a-c,
- Figures 16a-b: show side views of a bending actuator according to one embodiment of the invention,
- Figures 17a-b: schematically iiiustrate the operation of a segment in a device according to one embodiment of the invention which includes actuators of the type shown in the figures 16a-b,
- Figures 18-19: schematically illustrate devices according to embodiments of the invention, and
- Figures 20-23: illustrate examples of therapeutic garments including the proposed device.

### DESCRIPTION OF PREFERRED EMBODIMENTS OF THE INVENTION

The invention is based on electroactive materials (EAM), such as electroactive polymers (EAP) and electroactive ceramics (EAC). The area of electroactive materials can be categorized into two major groups: field activated EAMs and ionic EAMs. In turn, the field activated EAMs include piezoelectric, electrostrictive and Maxwell stress activated materials, electrets, percolative composites and liquid crystal elastomers. On the other hand, ion-exchange polymer metal composite materials (IPMC), conducting polymers, carbon nanotubes and ion-exchange gels constitute examples of ionic EAMs.

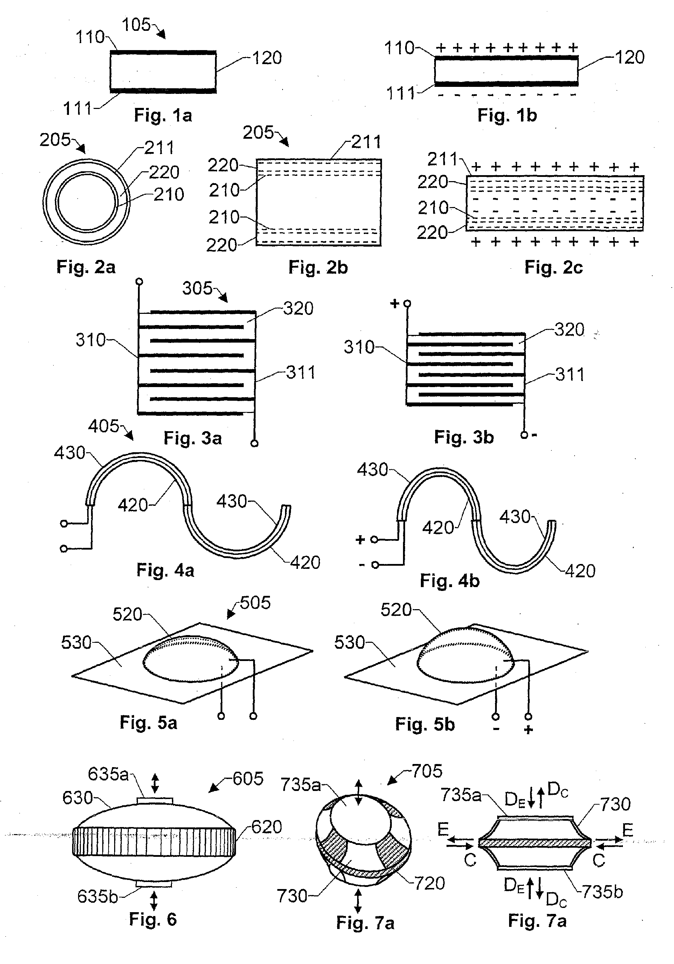

Figure 1a illustrates the basic morphology of a planar field activated EAM-based actuator 105. Two essentially plate-shaped electrodes 110 and 111 are here separated by means of an EAM piece 120. When an electric field is applied over the EAM piece 120, i.e. when one of the electrodes 110 is connected to a first polarity, say a positive voltage, and the other electrode 120 is connected to a second polarity, say a negative voltage, the EAM piece 120 undergoes a shape change, for instance by becoming thinner and longer. This situation is shown in figure 1b.

The shape change of the EAM 120 arises due to a variety of physical reasons when a non-zero charge is supplied to the electrodes 110 and 111, for example via a power supply or a control signal. In response to such a charge, the EAM 120 attempts to undergo a change in shape. The magnitude of the shape change depends on the material properties of the EAM 120, the frequency of the charge application/removal and mechanical boundary conditions of the material. Typically, the change in shape is related to the amount of charge accumulated on the surrounding electrodes 110 and 111.

In all dielectric materials, charge accumulation on adjacent electrodes creates mechanical stress in the material due to attraction and repulsion of the adjacent charges. Such stresses are referred to as Maxwell stresses. In soft materials, e.g. dielectric elastomers and gels, these stresses are sufficient to cause a significant shape change of the material.

Also in crystallographic materials, such as ceramics and ferroelectric polymers, an appreciable change in properties occurs with shape change of the material. This phenomenon is referred to electrostriction. Due to electrostrictive effects, the material will strive at changing the shape in response to an applied charge (in addition to Maxwell stress effects). Furthermore, an initial polarization may be frozen into some EAMs during manufacture. In materials capable of maintaining an initial polarization, the applied charge elicits a change in shape referred to as the reverse piezoelectric effect. Piezoelectric effects generally demonstrate a linear relationship between material strain and resulting electric field. Electrostrictive effects, on the other hand, generally demonstrate a quadratic relationship between material strain and applied electric field under linear boundary conditions. Under certain circumstances, these effects are reversible. Therefore, when the electroactive material undergoes a shape change, an electrical response occurs. This allows electric energy to be captured from the moving electroactive material. It also enables for the materials to operate as sensors.

Another important property of the EAMs is that a deformation (i.e. a changed morphology) resulting from an applied charge will be maintained if the electrodes are left open-circuited. Nevertheless, due to slight conductive effects, the thus separated charges will slowly leak through the EAM. Therefore, in practice, some replenishment/maintenance charge is necessary to top up the existing charge, and maintain a desired deformation.

Figures 2a and 2b illustrate top- and side views respectively of the morphology of a cylindrical field activated EAM-based actuator 205, which may be used to apply pressure according to the invention. Here, a first cylindrical electrode 210 is enclosed by an EAM piece 220. A second electrode_ 211, in turn, encloses the EAM piece 220. Figure 2c shows a side-view corresponding to the figure 2b, however where the first electrode 210 is connected to a positive voltage and the second electrode 211 is connected to a negative voltage. In similarity with the example shown in figure 1b, the EAM piece 220 contracts in response to the applied electric field, and the actuator's 205 diameter decreases while its length increases.

A multilayer cylindrical actuator of the type shown in the figures 2a and 2b may be accomplished straightforwardly by wrapping a planar actuator around a spring, or tube-like mandrel. Thereby, a compact, multilayered (i.e. high strength) tubular actuator can be cost effectively created from a simple planar starting geometry.

Figure 3a shows a schematic side-view of a field activated EAM-based actuator 305 of multilayer (or stacked) type, which may be used according to the present invention. Many interconnected layers of EAM 320 are here alternately separated by a first essentially planar electrode 310 and a second essentially planar electrode 311. Figure 3b illustrates the case when an electric field is applied across the electrodes 310 and 311. As can be seen, this again results in a contraction of the EAM 320. However, a material expansion may instead result if for example a piezoceramic is used as the EAM 320. In any case, by means of a stacked-type of actuator, a substantial mechanical amplification can be achieved. Triple layer actuators (or so-called bimorph cantilever actuators) can be formed by means of two EAM pieces separated by a supporting element, where opposite electric fields are applied to the EAM pieces. Thereby, the actuator can be controlled to bend in two different directions depending on which EAM piece that is activated, or the polarities applied to each of the EAM pieces.

Moreover, according to the invention, layers of passive materials may also be laminated along with the active material. These extra layers are often useful when interfacing with the surroundings, improving adhesion between adjacent active material layers, and creating favorable residual stresses in the active material during manufacturing.

Figure 4a shows a schematic side view of a field activated EAM-based actuator 405 of C-block type, which also may be used according to the invention. In each block of this actuator 405 a curved-profile laminate material 430 adjoins an EAM piece 420, which likewise has a curved profile. The general curved profile of each block amplifies the motion of the basic movement of the EAM piece 420. Two or more of these blocks may be connected in series with one another to accomplish a further amplification effect. When an electric field is applied between the respective EAM piece 420 and the laminate material 430, the EAM piece 420 contracts according to what is illustrated in figure 4b.

Figure 5a illustrates the morphology of a field activated EAM-based actuator 505 of bubble type according to one embodiment of the invention. Here, an EAP membrane 520, shaped as a truncated sphere (or similar bubble-like shape), is attached to a rigid mounting material 530. According to one embodiment of the invention, a pneumatic pressure bias is used to create the convex shape of the EAP membrane 520. An electric field across the EAP membrane 520 causes the membrane 520 to expand from its initial (inactivated) morphology. Figure 5b illustrates such an activated state. For example, such actuators can be used to create localized pressure points on the body.

Figure 6 shows a field activated EAM-based actuator 605 of cymbal type according to one embodiment of the invention. Multilayered EAM strips 620 are here located between two flexible, cymbal-shaped interface elements 630. When activated, i.e. in response to an electric field across the EAM strips 620, the strips 620 contract or expand and pull/push the interface elements 630 inwards or outwards as indicated by the arrows. Thereby, a pressure can be applied via first and second contact surfaces 635a and 635b respectively, which are located at the distal ends of the interface elements 630. Preferably, the interface elements 630 are flexible and the EAM strips 620 are oriented with their longest sides parallel to a symmetry axis of the interface elements 630, i.e. according to a layered structure as illustrated in the figure 6.

Figure 7a shows a perspective view of another cymbal type of field activated EAM-based actuator 705, which may be used in a device according to the invention. Figure 7b shows a sectional side view of this actuator 705. Here, two interface surfaces 735a and 735b are interconnected by means of a number of flexible members 730, which in turn, are attached to an EAM piece 720 located between the interface surfaces 735a and 735b. Thereby, upon activation of the actuator 705, so that the EAM piece 720 expands E, the interface surfaces 735a and 735b move toward one another D_{E} (essentially along their symmetry axes). Analogous thereto, upon activation of the actuator 705, so that the EAM piece 720 instead contracts C, the interface surfaces 735a and 735b are separated from one another D_{c} (essentially along their symmetry axes). Hence, a desired pressure can be created towards a body part through transformation of the basic material movement of the EAM piece 720.

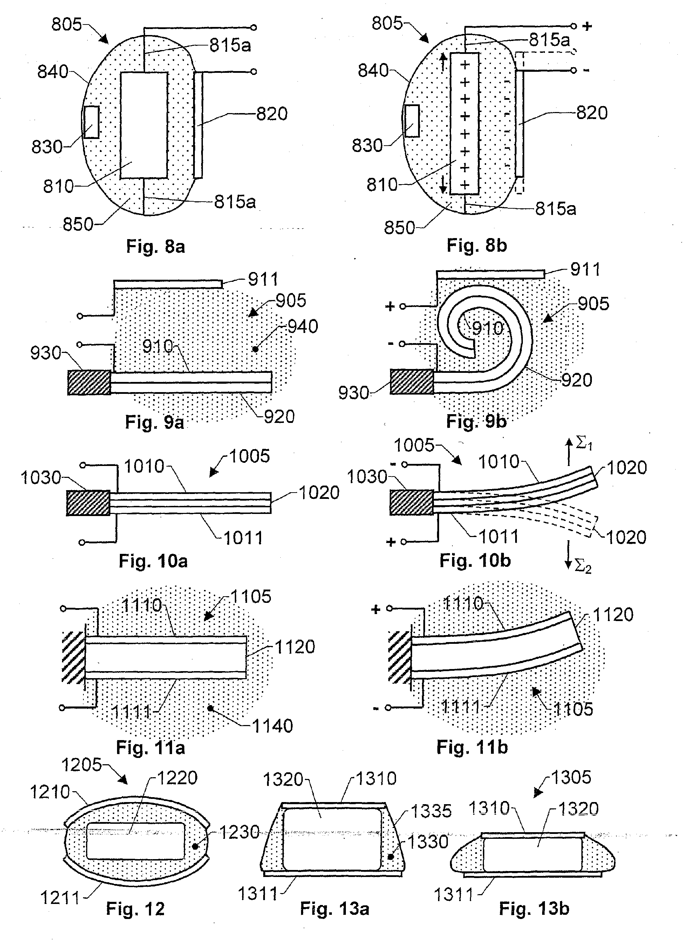

Figure 8a illustrates a basic morphology of an ionic EAM-based actuator 805 according to one embodiment of the invention. Ionic electroactive materials are characterized in that actuator systems based on them contain ions and that migration of these ions occurs under the influence of voltage potentials applied between electrodes 810 and 820 within the system. The ion migration, in turn, causes swelling or shrinking of the actuator. There are many designs based on the concept of electrically induced ionic migration. Some exemplary designs, which may be used according to the invention will be discussed below with reference to figures 8 to 13.

In similarity with field activated EAMs, ionic electroactive material reactions are reversible. Therefore, actuators based on ionic EAMs can also be used as various types of sensors and energy accumulators.

Returning now to figure 8a, conducting polymer actuators generally have an ion reservoir, such as an electrolyte 850 (in the form of a liquid, a gel or a solid), which separates a working electrode 810 and a counter electrode 820. The working electrode 810 usually includes the EAM (i.e. the conducting polymer). Also the counter electrode 820 may include an EAM, however usually different from the EAM of the working electrode 810. The counter electrode 820 may thus include a naturally conducting material, such as a metal or a graphite film. Moreover, the counter electrode 820 may be made from nanocomposites, fabricated to have both high conductivity and low mechanical stiffness.

The working electrode 810 may likewise include a composite of active materials and passive materials. In such cases, the passive materials are normally included to improve the conductive properties of the working electrode 810 while not impeding movement of the electrode during operation. Also here, conductive nanocomposites represent a viable option for lamination with the conducting polymer material.

In some cases, a reference electrode 830 is present in the electrolyte 850 to ensure that desired voltage potentials are maintained at appropriate levels at the other electrodes 810 and 820 during operation of the actuator 805 When the voltage potentials of the electrodes 810 and 820 are varied, the electroactive polymer can undergo oxidation or reduction reactions. Large electric fields are generated at the interfaces between the electrodes 810 and 820 respectively and the electrolyte 850. This causes ion migration across the interfaces. Ions within the EAM can initiate conformational change of the crystallographic structures of the material, or they may take up interstitial spaces in the material and cause it to swell. If ions are extracted from the EAM in the working electrode 810 due to migration, the working electrode 810 may shrink. Depending on the counter electrode material, reactions at the electrode 820 may or may not result in another usable shape change in respect of this electrode. The details of the entire actuator system (such as the specific EAMs, electrolyte and counter electrode properties) together dictate the final response of the system during operation.

Figure 8b illustrates a situation where a positive voltage has been applied to the working electrode 810 and a negative voltage has been applied to the counter electrode 820. As a result, the working electrode 810 extends while its width decreases. For improved expansion properties, the working electrode 810 may be designed as a composite of conductive helical supporting structure encapsulated by the EAM (not shown here). A possible extension of the counter electrode 820 (resulting from an included EAM) is illustrated by means of a dashed profile.

In the case of carbon nanotubes, the presence of carbon nanotubes dramatically increases the surface area of the electrode. This, in turn, increases the strength of the electromagnetic field during a reduction or oxidation process. Consequently, an increased ion migration occurs and therefore an amplified mechanical response can be obtained. The formation of gas can arise intimately with the electrode during a reaction (often due to electrolysis of water). Of course, the expansion of such gases may also result in an increased response.

Figure 9a illustrates the morphology of a bilayer ionic EAM-based actuator 905 according to one embodiment of the invention. Here, a working electrode 910 of a conductive polymer (EAP) adjoins a non-conducting polymer backing element 920, which mainly functions as a mechanical support to the actuator laminate of the working electrode 910. A counter electrode 911 is arranged physically separated from both the working electrode 910 and the backing element 920. The working electrode 910 and the EAP 920 are attached to an anchor member 930, and all the elements 910, 920 and 911 are surrounded by an electrolyte 940. Figure 9b shows a situation when a negative voltage has been applied to the working electrode 910 and a positive voltage has been applied to the counter electrode 911, causing the conductive polymer to contract, and as a result, the entire working electrode and backing element system to bend.

Figure 10a illustrates the morphology of a triple ionic layer EAM-based actuator 1005 according to one embodiment of the invention, which is similar in morphology to the actuator design of figures 9a and 9b. Here however, two conductive polymer electrodes 1010 and 1011 are separated by means of a non-conducting polymer element 1020. All the elements 1010, 1011 and 1020 are attached to an anchor member 1030, and may or may not be surrounded by an electrolyte. Namely, the non-conducting polymer element 1020 may include a solid polymer electrolyte, and thus forego the need for a surrounding electrolyte. in such a case, the elements 1010, 1011 and 1020 must be encapsulated to prevent evaporation of the electrolyte. Figure 10b illustrates how the actuator 1005 upon activation can be controlled to flex in different directions Σ₁ and Σ₂ depending on the polarity of a voltage applied between the electrodes 1010 and 1011. In this case, the actuator 1005 bends upwards Σ₁ in response to a negative voltage potential connected to the first electrode 1010 and a positive voltage potential connected to the second electrode 1011. Correspondingly, the actuator 1005 would bend downwards Σ₂ in response to opposite voltage potentials. Figures 11a and 11b illustrate the morphology of an ion-exchange polymer metal composite (IPMC) EAM-based actuator 1105 according to one embodiment of the invention. In this type of actuators electric fields generated in a polymer element 1120 causes cation migration in this polymer. A film (or laminate) of the actuator 1105 contains the polymer element 1120 adjoined by a first electrode element 1110 on one side and a second electrode element 1111 on the other side. All the elements 1110, 1111 and 1120 are surrounded by an electrolyte 1140 and are preferably attached to at least one anchor element. The electrode elements 1110 and 1111 may be made of a noble metal. During activation of the actuator 1105, migration of water molecules from the first electrode 1110 to the second electrode 1111 occurs, resulting in expansion of one side of the film and shrinking of the other side of the film. Consequently, the IPMC shows a bimorph-like motion. In this case, the electrolyte 1140 functions as an ion reservoir to refresh the actuator 1105 and keep it operable over time.

Ionic species may also take the form of a mobile plasticizer within the central polymer of the IPMC. Then, on application of electrode potential, ions will migrate to one electrode of the IPMC, resulting in uneven swelling of the laminate and hence bending motion. In this case, a surrounding electrolyte is not necessary and the actuator may operate in dry environments (i.e. here the actuator design is essentially the same as illustrated in the figures 11a and 11b, however without the electrolyte 1140).

Figure 12 shows an EAM-based actuator 1205, which includes an ion-exchange gel according to one embodiment of the invention. Here, a gel EAM 1220 is surrounded by an electrolyte 1230, which in turn, has interfaces towards first and second electrodes 1210 and 1211 respectively. When an electric potential is applied between the electrodes 1210 and 1211, the gel EAM 1220 changes its shape due to the expulsion or inclusion of hydrogen ions. Thus, a desired actuation of the actuator 1205 is accomplished. The principal difference between this type of ionic EAM and conducting polymers is that the ion-exchange gel itself acts as the electrolytic medium of the actuator.

Figures 13a-b illustrate another example of an EAM-based actuator 1305, which includes a gel EAM 1320 and that may be employed according to the present invention. In this case, a flexible wall 1335 encloses the gel EAM 1320 and an electrolyte 1330. The electrolyte 1330 interfaces first and second electrodes 1310 and 1311 respectively. In response to an electric field across the electrodes 1310 and 1311, the gel EAM 1320 contracts, such that the electrodes 1310 and 1311 approach one other (see figure 13b).

In addition to the actuator morphologies shown in the figures 8 to 13, the actuators according to the invention may take the form of fibers, fabrics or strips based on the basic concept ionic EAM-based actuators. Naturally, according to the invention, the basic actuator morphologies may be combined and be arranged in arrays to produce more advanced actuators.

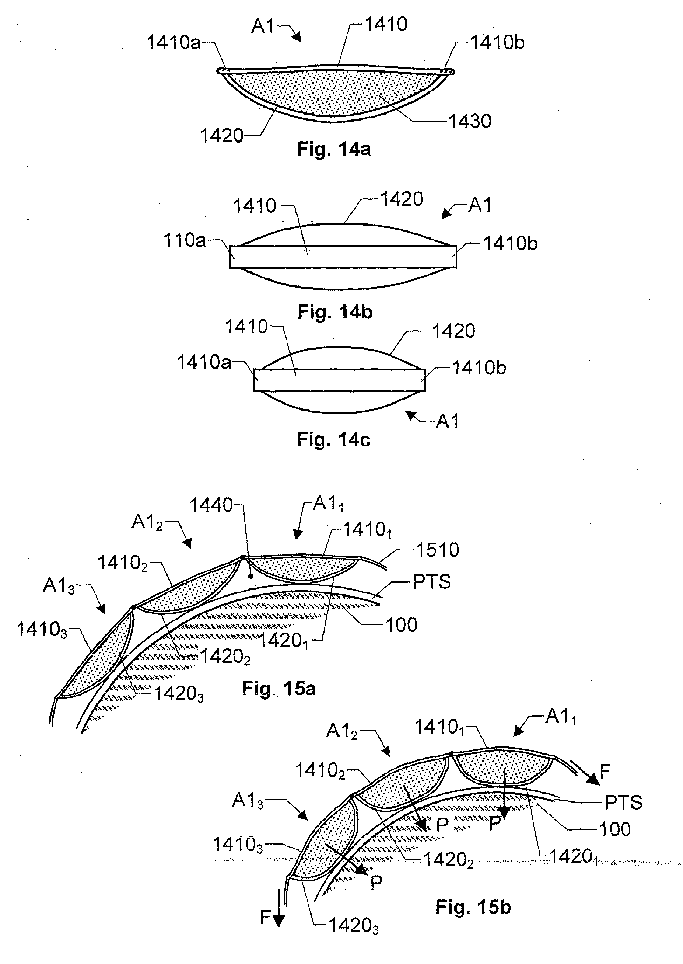

Figure 14a shows a side view of an inactivated conducting polymer actuator A1 according to one embodiment of the invention. Figure 14b shows a corresponding top view. This actuator A1 operates according to the basic principle described above with reference to the figures 8a and 8b. Nevertheless, both a working electrode 1410 and a counter electrode 1420 include a conductive polymer. An electrolyte 1430 is enclosed by these electrodes 1410 and 1420. The actuator A1 is activated by means of an applied voltage between a first electrode tab 1410a and a second electrode tab 1410b. When such a voltage is applied, the working electrode 1410 contracts and the counter electrode 1420 expands. As a result, the actuator A1 both contracts in plane and expands out of the plane according to the top-view illustration of figure 14c.

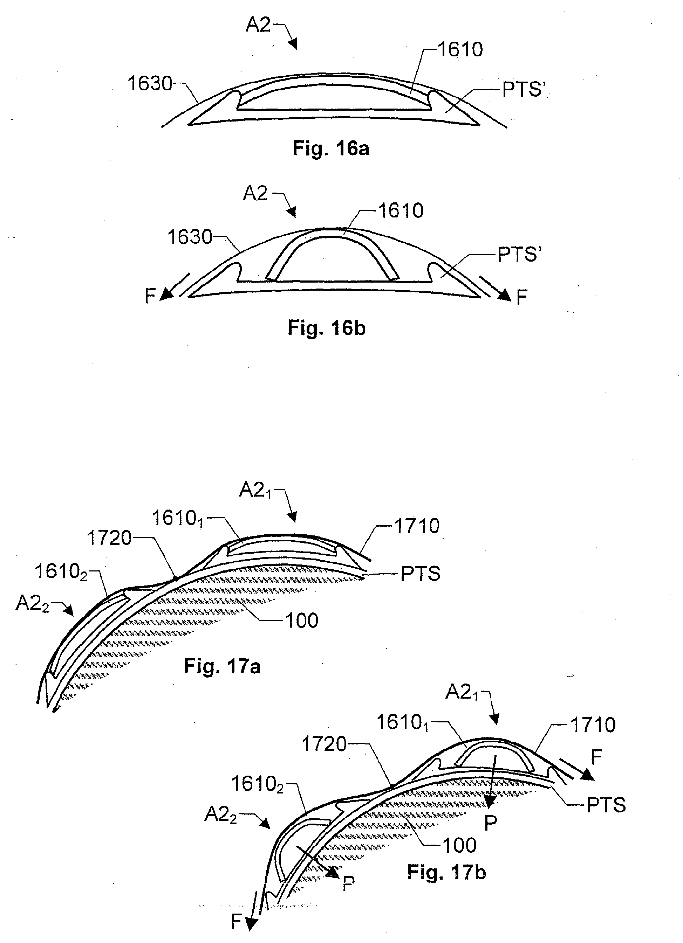

Figure 15a illustrates a side view of a segment in a device according to one embodiment of the invention, which includes a number of actuators A1₁, A1₂, and A1₃ of the type shown in the figures 14a-c. Theoretically, one actuator is sufficient to exert an external pressure to a body part 100 by means of the proposed device. However, for improved effect a plurality of actuators may be used. If so, the actuators are mechanically connected to one another to at least partially enclose the body part 100, such as the limb of a patient, in a form-fitting manner. Depending on the number of actuators and the range of motion of each actuator A1₁, A1₂ and A1₃, the device may be capable of completely disengaging the body part 100 at the conclusion of treatment. In this case, a strap 1510 (or equivalent) transmits actuator forces around the body part 100 and fixates the actuators A1₁, A1₂ and A1₃ to the body part 100. In other cases, the strap 1510 may contain a variety of locking mechanisms to assist with the removal of the device from the patient after treatment, or assist with size adjustment of the device to the patient.

Here, a first actuator A1₁ includes a first working electrode 1410₁ and a first counter electrode 1420₁; a second actuator A1₂ includes a second working electrode 1410₂ and a second counter electrode 1420₂; and a third actuator A1₃ includes a third working electrode 1410₃ and a third counter electrode 1420₃. Further, the first actuator A1₁ is connected to the second actuator A1₂, which in turn, is connected to the third actuator A1₂ according to the configuration of figure 15a. Strap members 1510 are attached to the first and third actuator A1₁ and A1₃ to fixate the device to the body part 100. The electrode tabs of each actuator A1₁, A1₂, and A1₃ are also electrically connected to an electric power source, so that the actuators can be activated by means of electric charges being supplied to their electrodes. However, for reasons of a clear presentation, this is not shown in the figure 15a.

Preferably, a pressure transition system PTS is arranged as an interface between the actuators A1₁, A1₂ and A1₃ and the body part 100. The pressure transition system PTS is adapted to redistribute a basic pressure profile of the actuators A1₁, A1₂ and A1₃, such that when the actuators are activated, an adjusted pressure profile different from the basic pressure profile is applied to the body part 100. Thereby, a smoother (or more fuzzy) pressure profile P can be applied to the body part, which is desirable in many medical applications.

According to another preferred embodiment of the invention, any voids between the actuators A1₁, A1₂ and A1₃ and the pressure transition system PTS are filled with an open-celled foam (not shown). Namely, this further assists in redistributing pressure from the actuators A1₁, A1₂ and A1₃ to the body part 100 without overly affecting the breath ability of the device.

Figure 15b illustrates a situation when all the actuators A1₁, A1₂ and A1₃ are activated, and therefore each actuator A1₁, A1₂ and A1₃ has adapted morphology equivalent to what is shown in the figure 14c. As a result, a pressure profile P is applied to the body part 100, and as a further consequence the body part 100 is normally compressed/deformed (which is here illustrated by means of a reduced cross-section diameter). If, however, the body part 100 were very stiff, and therefore would not deform under the pressure profile P applied, the actuators A1₁, A1₂ and A1₃ and the strap members 1510 would exert tensile forces F around the body part 100 without undergoing the large deformations depicted in the figure 15b.

Figures 16a and 16b show side views of a bending actuator A2 according to one embodiment of the invention. Preferably, this type of actuator A2 includes a bending member 1610 of field activated EAM-type. The bending member 1610 is adapted to operate against a local pressure transition system PTS' and an over layer 1630, for instance in the form of an appropriate interfacing fabric. An elastic back plate side of the bending member 1610 faces the over layer 1630. Figure 16a illustrates an inactivated state of the actuator A2, while figure 16b illustrates an activated state. As can be seen, when activated the bending member 1610 pushes the over layer 1630 away from the local pressure transition system PTS'. This leads to tensile forces F in the over layer 1630, which according to the invention may be converted into a desired pressure profile applied to a body part.

Figure 17a shows a side view of a segment in a device according to one embodiment of the invention, which includes a number of actuators A2₁ and A2₂ of the type shown in the figures 16a and 16b. In the configuration shown, the number of actuators is increased to accommodate a wider range of available motion, and to more evenly distribute pressure to a curved body part 100. If an increase pressure is desired, two or more actuators may be applied in parallel, i.e. be stacked, such that the actuators a layered on top of one another. Alternatively, multilayered laminates within each actuator element of an actuator array may be thickened. Nevertheless, for illustrative purposes only two actuators A2₁ and A2₂ are shown here. The actuators A2₁ and A2₂ are located next to one another and have a common over layer 1710 (compare with 1630 in the figures 16a and 16b). Preferably, the over layer 1710 has at least one attachment point 1720 between the actuators A2₁ and A2₂. Thereby, when activated, each actuator A2₁ and A2₂ contributes maximally to the generation of a pressure towards the body part 100. Moreover, in addition to the local systems of each actuator, a pressure transition system PTS is preferably arranged as an interface between the actuators A1₁ and A1₂ respectively and the body part 100. Such a pressure transition system PTS is adapted to redistribute a basic pressure profile of the actuators A1₁ and A1₂, so that when the actuators are activated, an adjusted pressure profile different from the basic pressure profile is applied to the body part 100. Thereby, a smoother (or more fuzzy) pressure profile P can be applied to the body part, which is desirable in many medical applications.

In similarity with the figure 15b, figure 17b shows a situation when the actuators A1₁ and A1₂ are activated, and a pressure profile P is applied to the body part 100. Normally, this leads to a compression/deformation of the body part 100 (which is here illustrated by means of a reduced cross-section diameter). If, however, the body part 100 were very stiff, and therefore would not deform under the pressure profile P applied, yet the over layer 1710 would still exert tensile forces F around the body part 100.

For proper function, the outer layer 1710 should be made of a rigid and strong fabric, so that it does not stretch appreciably during activation of the actuators A2₁ and A2₂. Therefore, the outer layer 1710 is preferably a knitted anisotropic material, which includes fibers (e.g. of Kevlar) that are strong in the circumferential direction (i.e. around the body part 100), and relatively soft in the axial direction (i.e. along the body part 100). Moreover, the outer layer 1710 fabric preferably has an open weave to ensure that the breath ability of the device is not compromised.

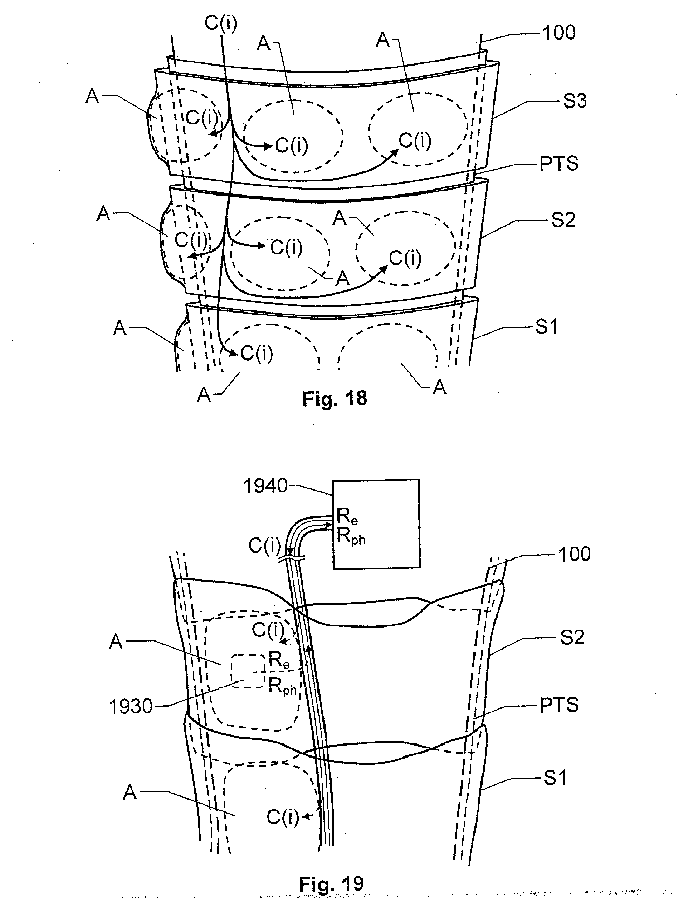

Figure 18 shows a side view of a device according to one embodiment of the invention for exerting an external pressure to a human body part 100. The device includes segments S1, S2 and S3, which are adapted to at least partially enclose the body part 100 in a form-fitting manner. Each segment S1, S2 and S3 contains a controllable active-material based actuator A (e.g. of the type illustrated in the figures 17a and 17b) that is adapted to cause the segment to apply a pressure profile to the body part 100 in response to a control signal C(i). The device may also include a pressure transition system PSS, which is adapted to redistribute a basic pressure profile produced by the segments S1, S2 and S3 into an adjusted pressure profile which is different from the basic pressure profile. Thus, the pressure transition system PSS accomplished a relatively smoothed-out, or fuzzy, pressure profile to be applied to the body part 100. This is advantageous both from a medical and a patient-comfort point-of-view. The pressure transition system PSS, in turn, preferably includes an auxetic-foam composite or other material having a deformable microcellular structure.

Figure 19 shows a side view of a device according to another embodiment of the invention for exerting an external pressure to a human body part 100. Here, each of a number of segments S1, S2, etc. at least partially encloses a body part 100. Moreover, the segments are arranged such that a portion of one segment S1 covers a portion of a neighboring segment S2, and so on. Thereby, analogous to the embodiment described above with reference to figure 18, relatively smoothed-out, or fuzzy, pressure profiles may be applied to the body part 100 in response to control signals C(i) in respect of the segments S1 and S2 also without a pressure transition system. In similarity with the above-described embodiment, each segment S1 and S2 contains a controllable active-material based actuator A (e.g. of electroactive ceramics or polymer, conducting-polymer, carbonnanotube or electroactive-gel type)

According to this embodiment of the invention, at least one segment, say S2, includes a sensor element 1930, which is adapted to register relevant parameters, and transmit data signals Rₚₕ, Rₑ reflecting these parameters to a control unit 1940 for analysis. For example, the sensor element 1930 may be a thin film force sensor (e.g. capacitive, piezoresistive, piezoelectric, varying contact or Quantum Tunneling Composite - QTC) adapted to register pressure. The sensor element 1930 may also be intended to monitor the local circumference of the body part 100. In this case, the element 1930 is preferably a resistive strip, an interdigitated electrode with contacts, or similar sensor which surrounds the body part 100. Additionally, the sensor element 1930 may be responsible for measuring physiological parameters, such as heart rate, galvanic skin response, electromyogram - EMG, blood oxygen levels, exudates extraction rates all of which are represented by a first type of data signals Rₚₕ. Moreover, the sensor element 1930 may be adapted to register a parameter expressing an environmental condition in proximity to the body part 100, e.g. temperature, airflow, humidity or contamination, which may be represented by a second type of data signals Rₑ. Namely, these types of environmental conditions may also influence what is an ideal behavior of the proposed device. Thus, based on at least one of the first and second types of data signals Rₚₕ and Rₑ respectively from the sensor element 1930, a treatment profile executed by the device may be adjusted, so that the treatment profile depends on a current state of the body part 100 and/or the current environmental conditions. The treatment profile may involve applying gradually varying pressure profiles to the body part 100 via the segments S1 and S2. Particularly, the treatment profile controlled by the control unit 1940 may entail producing repeated cycles of variations between relatively high and relatively low basic pressure profiles. Alternatively, the treatment profile could involve producing quasi-static pressure profiles by means of the segments S1 and S2.

Moreover, the data signals Rₑ, Rₚₕ may reflect a patient's posture. Hence, according to the invention, it is rendered possible to adapt the treatment profile to the posture. For example, if the segments S1, S2, ..., Sn are fitted around the patient's leg, they may be completely relaxed when the patient is lying down (e.g. apply a pressure profile in the range 0-10 mmHg), apply a relatively low graduated pressure profile (e.g. in the range 0-40 mmHg) when the patient is standing up and apply a relatively high graduated pressure profile (e.g. in the range 0-60 mmHg) when the patient is sitting.

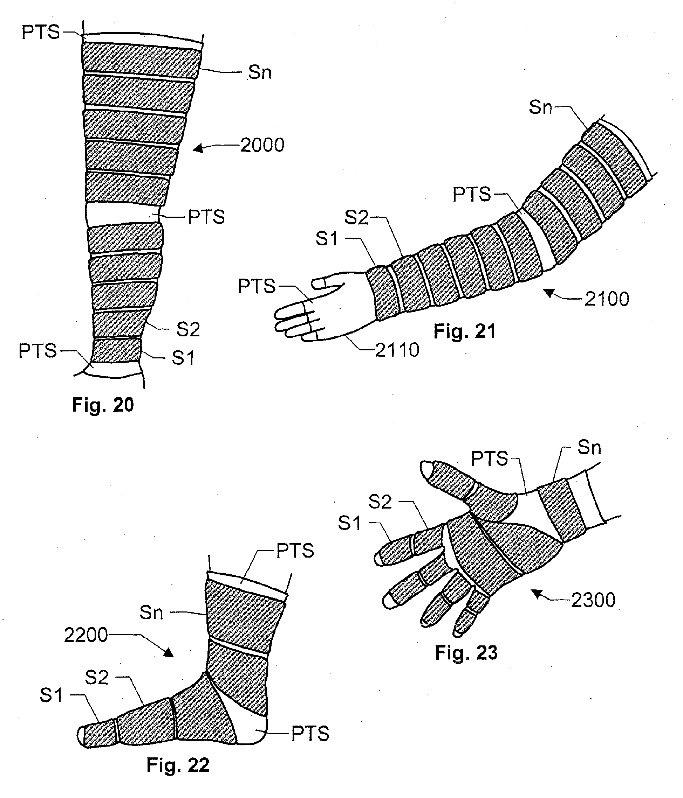

Figures 20-23 illustrate examples of therapeutic garments 2000, 2100, 2200 and 2300 including the proposed device.

Figure 20 shows a leg garment 2000, where a plurality of segments S1, S2, ..., Sn are adapted to enclose both the upper and the lower part of a patient's leg. An extensive pressure transition system PSS is here adapted to redistribute basic pressure profiles generated by the segments S1, S2,...,Sn, so that the entire leg is exerted to adjusted pressure profiles, for example also at joint portions of the leg that are not covered by any segments.

Figure 21 shows an arm garment 2100, where a plurality of segments S1, S2, ..., Sn are adapted to enclose a patient's forearm and over arm. Also here an extensive pressure transition system PSS is adapted to redistribute basic pressure profiles generated by the segments S1, S2, ..., Sn. Thereby, for instance, an elbow portion which for flexibility reasons is not covered by segments may be exerted to pressure. Additionally, the pressure transition system PSS is adapted to extend over the patient's hand in the form of a compression glove, so as to prevent lymphatic fluids from pooling in the hand.

Figures 22 and 23 show garments 2200 and 2300 for exerting external pressures to a patient's foot and hand respectively. In both these cases a plurality of segments S1, S2, ..., Sn are adapted to enclose a said extremities, and a pressure transition system PSS is adapted to redistribute basic pressure profiles generated by the segments S1, S2, ..., Sn. Thus, basic pressure profiles may be smoothed out, and also portions which are not covered by segments may be pressurized.

The term "comprises/comprising" when used in this specification is taken to specify the presence of stated features, integers, steps or components. However, the term does not preclude the presence or addition of one or more additional features, integers, steps or components or groups thereof.

The invention is not restricted to the described embodiments in the figures, but may be varied freely within the scope of the claims.

## Claims

1. A device for exerting an external pressure to a human body part (100), the device comprising a number of segments (S1, S2, S3) adapted to at least partially enclose the body part (100) in a form-fitting manner and apply an adjustable pressure profile to the body part (100), **characterized in that**
each segment (S1, S2, S3) contains an electroactive-material-based actuator (A1, A2, A) which is adapted to produce the adjustable pressure profile (P) in direct response to an electrical control signal (C(i)).

2. A device according to claim 1, **characterized in that**
each of said actuators (A) has a morphology which is adjustable by the electrical control signal (C(i)),
a change of said morphology is instigated by the electrical control signal (C(i)), and
each of said actuators (A) is adapted to maintain a thus changed morphology on the basis of an electrical control signal (C(i)) supplying charge replenishment to the actuator (A).

3. A device according to any one of the preceding claims, **characterized in that** at least one of said actuators (A1, A2, A) is adapted to convert mechanical energy produced by the body part (100) into electric energy.

4. A device according to any one of the preceding claims, **characterized in that** at least one of said segments (S1, S2, S3) comprises an actuator (A2; A2₁, A2₂; A) that has an interface towards a pressure transition system (PTS', PTS) adapted to redistribute a basic pressure profile of the actuator, such that in response to a control signal (C(i)) in respect of the segment an adjusted pressure profile different from the basic pressure profile is applied to the body part (100).

5. A device according to any one of the preceding claims, **characterized in that** at least one of said actuators (A1, A2, A) comprises an electroactive polymer.

6. A device according to any one of the preceding claims, **characterized in that** at least one of said actuators (A1, A2, A) comprises an electroactive ceramic.

7. A device according to any one of the claims 5 or 6, **characterized in that** at least one of said actuators (105, 205, 305, 405, 505, 605, 705) comprises a field activated electroactive material (120; 220; 320; 420; 520; 620; 720).

8. A device according to claim 7, **characterized in that** at least one of said actuators (105, 205, 305, 405, 505, 605, 705) is adapted to operate based on at least one of Maxwell stress effects, electrostrictive effects and piezoelectric effects.

9. A device according to any one of the claims 5 - 8, **characterized in that** at least one of said actuators (805, 905, 1005, 1105, 1205, 1305) comprises an ionic electroactive material (820; 920; 1020; 1120; 1220; 1320).

10. A device according to claim 9, **characterized in that** at least one of said actuators (805, 905, 1005, 1105, 1205, 1305) comprises at least one of an ion-exchange polymer metal composite material and a conducting polymer.

11. A device according to any one of the claims 9 or 10, **characterized in that** at least one of said actuators (1205, 1305) comprises a gel-based electrolyte (1220, 1340).

12. A device according to any one of the preceding claims, **characterized in that** at least one of said actuators (605, 705) comprises at least one flexible interface (630, 635a, 635b; 730, 735a, 735b) towards the segment to which the actuator is associated, said at least one flexible interface (630, 635a, 635b; 730, 735a, 735b) is adapted to link mechanical energy produced the actuator (605, 705) towards the body part (100).

13. A device according to any one of the preceding claims, **characterized in that** the device comprises a control unit (1940) adapted to apply control signals (C(i)) to said segments (S1, S2), and the control unit (1940) is adapted to vary the control signals (C(i)) over time to implement a treatment profile with respect to the body part (100).

14. A device to any one of the preceding claims, **characterized in that** the treatment profile involves applying gradually varying pressure profiles (P) to the body part (100) via said segments (S1, S2, S3).

15. A device according to claim 14, **characterized in that** the treatment profile involves producing repeated cycles of variations between relatively high and relatively low basic pressure profiles (P) by means of each of said segments (S1, S2, S3).

16. A device according to claim 13, **characterized in that** the treatment profile involves producing quasi-static pressure profiles (P) by means of said segments (S1, S2, S3).

17. A device according to any one of the claims 13 - 16, **characterized in that** at least one of said actuators (A) comprises a sensor element (1930) adapted to register a physiological parameter of the body part (100), and generate a data signal (Rpₕ) reflecting this parameter.

18. A device according to any one of the claims 13 - 17, **characterized in that** at least one of said actuators (A) comprises a sensor element (1930) adapted to register a parameter expressing an environmental condition in proximity to the body part (100), and generate a data signal (Rₑ) reflecting this parameter.

19. A device according to any one of the claims 17 or 18, **characterized in that** the sensor element (1930) constitutes a subdivision of one of said actuators (A).

20. A device according to any one of the claims 17 - 19, **characterized in that** the control unit (1940) is adapted to receive at least one of said data signals (Rₚₕ, Rₑ) from at least one of said at least one sensor element (1930), and adapt the treatment profile in response to said at least one data signal (Rₚₕ, Rₑ).

21. A therapeutic garment (2000, 2100, 2200, 2300) adapted to cover at least one appendage of a human body, **characterized in that** the garment comprises at least one device according to any one of the claims 1 - 20.
